(19) Europäisches Patentamt
European Patent Office
Office européen des brevets

(11) Numéro de publication: **0 046 720**
**B1**

(12) **FASCICULE DE BREVET EUROPEEN**

(45) Date de publication du fascicule du brevet: **08.08.84**

(51) Int. Cl.³: **C 08 G 59/06**

(21) Numéro de dépôt: **81420125.7**

(22) Date de dépôt: **24.08.81**

(54) **Procédé de préparation de polyéthers glycidiques de polyphénols.**

(30) Priorité: **27.08.80 FR 8018563**

(43) Date de publication de la demande: **03.03.82 Bulletin 82/9**

(45) Mention de la délivrance du brevet: **08.08.84 Bulletin 84/32**

(84) Etats contractants désignés: **AT BE CH DE FR GB IT LI LU NL SE**

(56) Documents cités:
**EP-A-0 013 532**
**EP-A-0 015 860**

(73) Titulaire: **RHONE-POULENC CHIMIE DE BASE, 25, quai Paul Doumer, F-92408 Courbevoie (FR)**

(72) Inventeur: **Locatelli, Jean-Louis, Les Charavelles Bt G 10 Rue de Charavel, F-38200 Vienne (FR)**
Inventeur: **Soula, Gérard, 33, rue Nungesser, F-69330 Meyzieu (FR)**

(74) Mandataire: **Varniere-Grange, Monique et al, RHONE-POULENC RECHERCHES Service Brevets Chimie et Polymères Centre de Recherches de Saint-Fons B.P. 62, F-69192 St-Fons Cédex (FR)**

**EP 0 046 720 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation de polyéthers glycidiques de polyphénols, à partir de sels alcalins de polyphénols et de halo-1 époxy-2,3 alcanes.

Ces éthers sont plus couramment désignés par l'expression »résines époxydes«. Ces résines constituent une classe de produits caractérisés par la présence de cycles oxirannes

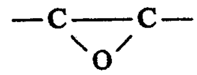

qui conduisent, après réticulation, à des systèmes dont les propriétés sont remarquables en divers points. Ceci a largement contribué au développement de ce type de résines, dans de nombreux domaines d'application.

Parmi ces résines, celles qui sont traditionnellement issues de la réaction du bisphénol-A [bis(hydroxy-4 phényl)-2,2 propane] et de l'épichlorhydrine présentent un intérêt tout particulier.

La présente invention a donc pour objet ur procédé de préparation d'éthers glycidiques, notamment du bisphénol-A par réaction en milieu anhydre et aprotique de sels alcalins de polyphénols et de halo-1 époxy-2,3 alcanes.

Un autre objet de l'invention est de fournir un procédé permettant de préparer des résines ayant une viscosité mesurée à 25° C inférieure ou égale à 150 poises (15 Pa . s).

Ce type de résines est conventionnellement préparé par réaction du bisphénol-A, sur de l'épichlorhydrine, en présence d'eau et d'un agent alcalin. De très nombreux procédés ont été décrits dans des travaux antérieurs. Ces procédés sont toutefois extrêmement lourds à mettre en œuvre: ils nécessitent des durées de reaction relativement longuer, des contrôles stricts des diverses conditions de réaction et de nombreuses étapes pour purifier et/ou récupérer la résine recherchée. D'autre part, les pertes en épichlorhydrine, engagée nécessairement en excès, ne sont pas négligeables même lorsque le plus grand soin est apporté à la mise en oeuvre de ce genre de procédé.

Très récemment, des auteurs ont envisagé une autre voie d'accès à ce type de résine. C'est ainsi (Cf. MAKROMOL. CHEM 179,7,1661—1671, 1978) qu'il a été proposé de réaliser la synthèse des résines en cause, à partir de sels alcalins de certains diphénols et de halo-1 époxy-2,3 alcanes, en milieu anhydre et aprotique.

Toutefois, la réaction est limitée par la difficulté à solubiliser les sels alcalins de diphénols. En effet, de grandes quantités de diméthylsulfoxide doivent être utilisées pour rendre le milieu homogène, ce qui entrave le développement d'une telle technique à l'échelle industrielle.

Il a maintenant été trouvé que l'on peut préparer des éthers glycidiques de polyphénols à partir de sels alcalins de polyphénols et de halo-1 époxy-2,3 alcanes en milieu sensiblement anhydre et aprotique, sans être forcément en milieu homogène, à condition d'opérer en présence de catalyseurs, définis ci-après.

Le procédé selon l'invention permet d'obtenir par une technique relativement simple et rapide, des résines époxydes présentant de très bonnes caractéristiques.

Selon l'invention, on fait réagir au moins un sel alcalin d'un polyphénol sur au moins un halo-1 époxy-2,3 alcane, en présence d'un catalyseur supporté constitué par un support polymère organique réticulé et par une pluralité de groupes fonctionnels (groupes actifs du catalyseur), fixés sur ledit support et ayant pour formule générale:

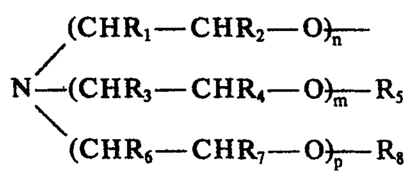
(I)

dans laquelle $R_1$, $R_2$, $R_3$, $R_4$ $R_6$ et $R_7$, identiques ou différents, sont chacun un atome d'hydrogène ou un radical alkyle ayant de 1 à 4 atomes de carbone, $R_5$ et $R_8$, identiques ou différents, représentent un atome d'hydrogène, un radical alkyle ou cycloalkyle ayant de 1 à 12 atomes de carbone un radical phényle ($C_6H_5—$), un radical $C_6H_5—C_qH_{2q}—$ ou $C_q\ H_{2q+1}—C_6H_5$ — avec q compris entre 1 et 12 ($1 \leq q \leq 12$), et dans laquelle n, m et p, identiques ou différents, sont supérieurs ou égaux à 1 et inférieurs ou égaux à 10.

Selon un mode de réalisation préférentiel de la présente invention les groupes fonctionnels du catalyseur sont choisis parmi les groupes de formule (I) ci-avant dans laquelle $R_1$, $R_2$, $R_3$, $R_4$, $R_6$ et $R_7$, identiques ou différents, représentent un atome d'hydrogène ou un radical méthyle et $R_5$ et $R_8$, identiques ou différents, représentent un atome d'hydrogène ou un radical alkyle ayant de 1 à 4 atomes de carbone.

Selon un autre mode de réalisation préférentiel de l'invention n, m et p, identiques ou différents, sont supérieurs ou égaux à 1 et inférieurs ou égaux à 6.

A titre d'exemples de groupes fonctionnels convenant à la mise en oeuvre du présent procédé on

peut citer les groupes de formules suivantes.

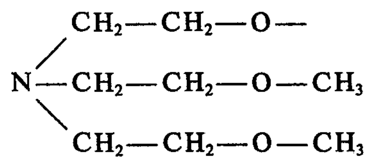

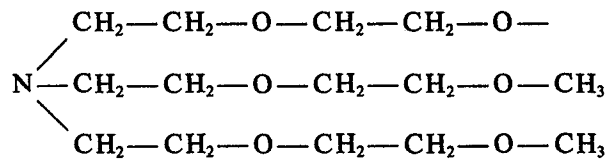

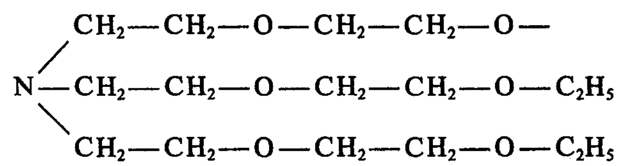

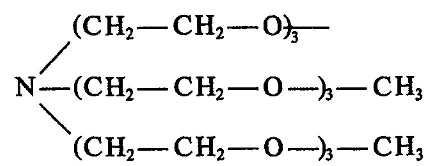

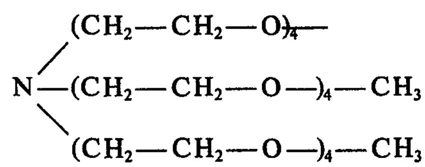

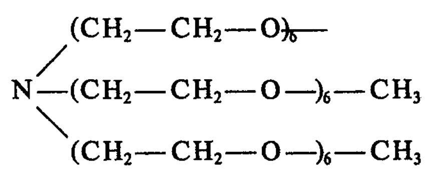

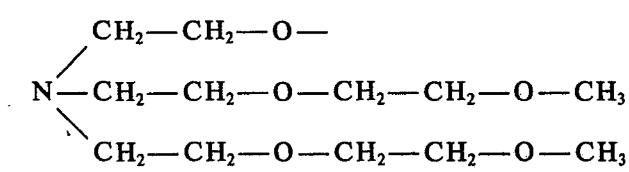

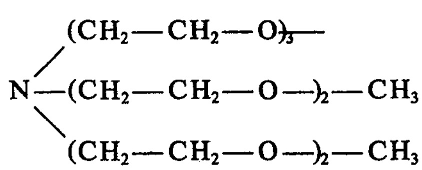

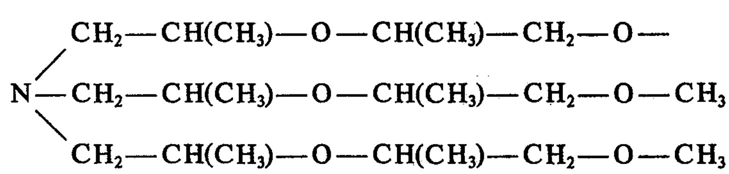

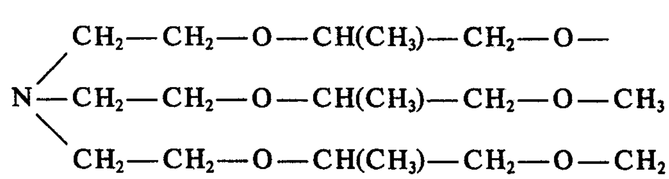

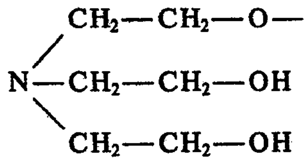

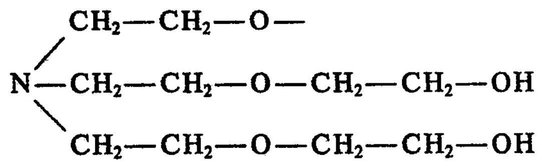

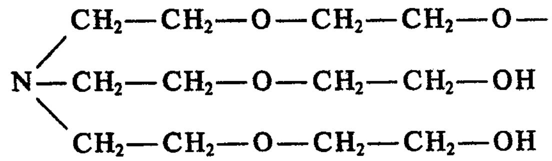

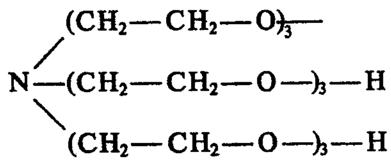

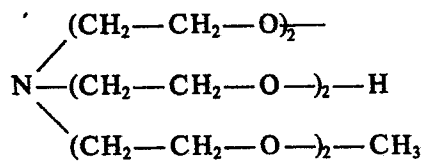

Comme indiqué ci-avant les groupes actifs du catalyseur sont fixés sur un support qui est un polymère organique réticulé. Ce support peut dériver de tout polymère organique réticulé comportant des groupements substituables par les groupements fonctionnels de formule I.

Des polymères adaptés à la préparation des catalyseurs mis en oeuvre dans le présent procédé sont, par exemple, les polymères dérivés de composés vinylaromatiques tels que le styrène, le méthylstyrène et les copolymères de composés vinylaromatiques et de diènes conjugués ayant de 4 à 6 atomes de carbone tels que les copolymères du styrène et du butadiène ou de l'isoprène.

Le polystyrène convient particulièrement bien comme polymère organique, l'agent de réticulation étant alors avantageusement le divinylbenzène. Le taux de réticulation est un facteur important. Il est en effet nécessaire que les groupes actifs du catalyseur, représentés par la formule I, gréffés sur le polymère soient accessibles. En d'autres termes, le taux de réticulation doit être suffisamment faible pour ne pas empêcher la pénétration au sein du polymère des molécules de réactifs et le cas échéant, d'un tiers-corps ou d'un solvant. A cette fin, la demanderesse préconise l'utilisation d'un polystyrène dont le taux de réticulation est inférieur à environ 10%, et de manière avantageuse, inférieur à 5%.

Parmi les groupements substituables appropriés on peut citer le chlore ou le brome du radical chloro ou bromo méthyle ($-CH_2Cl$ ou $-CH_2Br$) fixé sur le noyau benzènique du polystyrène.

Le pourcentage de noyaux benzèniques du polystyrène portant un groupement fonctionnel est de préférence supérieur à 5%, un pourcentage supérieur à 10% étant particulièrement avantageux. On peut représenter les catalyseurs supportés préférés par la formule suivante:

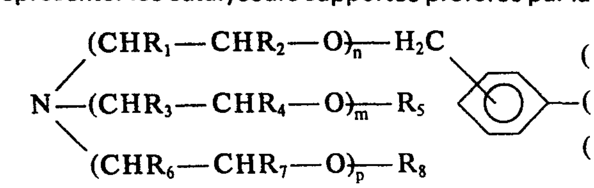

dans laquelle $R_1$ à $R_8$, n, m et p ont la signification donnée précédemment et,

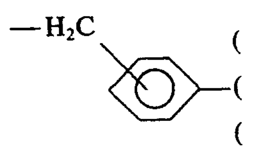

dérive du polystyrène chloro ou bromo méthylé de formule

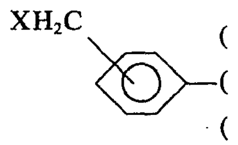

X représentant un atome de chlore ou de brome. Pour préparer les catalyseurs mis en oeuvre dans le présent procédé on fait réagir un composé de formule:

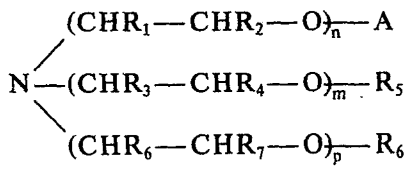
(II)

dans laquelle A représente un métal alcalin et $R_1$ à $R_8$, n, m et p ont la signification précédente avec un polymère organique réticulé comportant des groupements substituables tels que définis ci-avant, à une température de l'ordre de 20 à 150° C dans un solvant aprotique.

Une méthode commode de préparation comprend l'utilisation d'un composé de formule (II) dans laquelle A représente du sodium ou du potassium.

Une autre méthode plus particulièrement appropriée à ladite préparation comprend l'utilisation d'un solvant choisi parmi le groupe renfermant le benzène, le toluène, la N-méthylpyrrolidone, l'hexaméthylphosphorotriamide, le dioxane, le tétrahydrofuranne, le diméthoxyéthane et le sulfolane.

Selon une variante préférée, on fait réagir avec le composé de formule (II) un polystyrène chloro ou bromo méthylé ayant un taux de réticulation par le divinylbenzène inférieur à 10% et ayant un taux de chlore ou de brome compris entre 0,5 et 7 milliéquivalents de chlore ou de brome par gramme.

Des composés de formule (II) ci-avant peuvent être obtenus par exemple, par réaction du métal alcalin (par exemple du sodium métallique) en milieu solvant organique tel que le toluène, le tétrahydrofuranne, le dioxane etc. à une température comprise entre 60 et 90° C pendant 4 à 6 heures avec l'aminoalcool de formule:

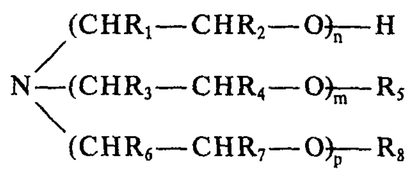
(III)

lui-même obtenu par réaction d'un polyalkylène glycol de formule:

$$HO-(CHR_1-CHR_2-O-)_n-H$$

dans laquelle $R_1$, $R_2$ et n ont la signification précédente, avec une bis(polyoxaalkylamine) de formule:

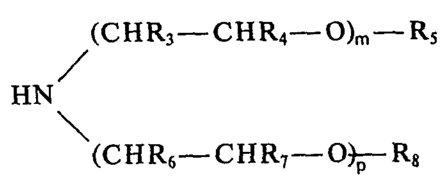

dans laquelle $R_3$ à $R_8$, m et p ont la signification précédente, le rapport molaire du polyalkylène glycol à la bis(polyoxaalkylamine) étant au moins égal à 1,5, en présence d'un catalyseur d'hydrogénation/deshydrogénation à une température comprise entre 120 et 220° C et, de préférence, entre 150 et 200° C. Comme catalyseur on peut utiliser des catalyseurs au nickel du type nickel Raney ou Harshaw, la quantité de catalyseur étant généralement comprise entre 1 et 15% en poids et, de préférence, entre 2 et 6%. Le rapport molaire du polyalkylène glycol à la bis(polyoxaalkylamine) est compris, de préférence, entre 1,5 et 10, et de manière encore plus avantageuse entre 2 et 6. La présence d'hydrogène sous pression autogène à raison de 1 à 10% (en poids) d'hydrogène par rapport au polyalkylène glycol mis en oeuvre, s'avère également avantageuse.

Les catalyseurs supportés constitués par un support polymère organique réticulé et par une pluralité de groupes fonctionnels (groupes actifs), fixés sur ledit support et correspondant à la formule (I) définie dans le présent mémoire, se sont révélés particulièrement efficaces dans la synthèse de polyéthers glycidiques de polyphénols, à partir de sels alcalins de polyphénols et de halo-1 époxy-2,3 alcanes.

Le procédé selon la présente invention nécessite la mise en oeuvre d'au moins un sel alcalin de polyphénol. Par sels alcalins de polyphénols, on attend des composés de formule:

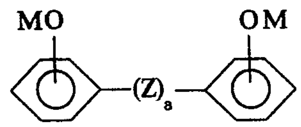

(IV)

dans laquelle a est égal à 0 ou 1, M représente un atome de lithium, de sodium, de potassium ou de césium, M étant de préférence un atome de sodium ou de potassium, et Z représente un radical divalent choisi dans le groupe comprenant les radicaux

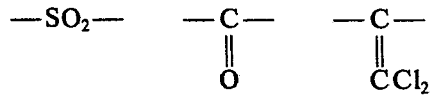

et les radicaux de formule (V) ci-après:

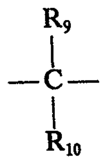

(V)

dans laquelle $R_9$ est un atome d'hydrogène ou un radical méthyle, $R_{10}$ représente l'hydrogène ou un radical monovalent choisi dans le groupe comprenant les radicaux $-CH_3$, $-CH_2-CH_3$, $-C(CH_3)_3$, $-CCl_3$,

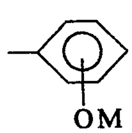

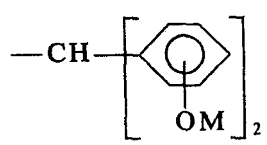

et

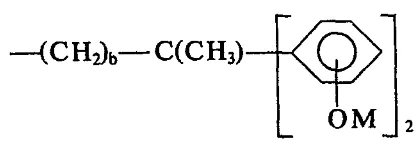

b étant égal à 0, 1 ou 2 et M ayant la signification précedente.

A titre d'exemple de polyphénols utilisable sous la forme de leurs sels alcalins, dans le cadre de la présente invention, on peut citer:

le dihydroxy-4,4′ diphényle
la dihydroxy-4,4′ diphénylsulfone
le dihydroxy-2,4′diphényl diméthyl méthane
le dihydroxy- 4,4′diphényl méthane (Bisphénol-F)
le bis(hydroxy-4 phényl)-1,1éthane
le bis (hydroxy-4 phényl)-1,1 isobutane
le bis (hydroxy-4 phényl)-2,2 propane (Bisphénol-A)
le bis (hydroxy-4 phényl)-2,2 butane
le bis (hydroxy-2 phényl)-2,2 propane
le bis (hydroxy-4 phényl)-2,2 trichloro-1,1,1 éthane
le bis (hydroxy-4 phényl)-2,2 dichloro-1,1 éthylène
le tris (hydroxy-4 phényl) méthane
le tetrakis (hydroxy-4 phényl)-1,1,2,2 éthane
le tétrakis (hydroxy-4′ phényl)-2,2,3,3 butane
le tétrakis (hydroxy-4′ phényl)-2,2,4, 4 pentane
le tétrakis (hydroxy-4′ phényl)-2,2,5,5 hexane

Le procédé selon l'invention, envisage également l'utilisation de sels alcalins de phénols polycycliques dans lesquels les atomes d'hydrogène du noyau ont été remplacés en partie par des atomes d'halogènes ou des radicaux alkyles. C'est le cas, par exemple, du bis (dibromo 3,5, hydroxy 4 phényl)-2,2 propane, du bis(dichloro-3,5, hydroxy-4 phényl)-2,2 propane, du bis(hydroxy 4, méthyl 2 phényl)-2,2 propane, du bis(hydroxy-2, tertiobutyl-4 phényl)-2,2 propane et du bis(chloro-2, hydroxy-4 phényl)-2,2 propane.

Le procédé selon l'invention envisage également l'utilisation de sels alcalins de phénols polycycliques plus complexes, tels que les résines novolaques. Ces résines sont obtenues par condensation, en

présence de catalyseurs acides, du phénol ou du crésol sur des aldehydes tels que le formaldéhyde, l'acétaldéhyde, le crotonaldéhyde, etc...

Selon la présente invention on peut utiliser des mélanges de deux ou plusieurs sels alcalins de polyphénols, c'est-à-dire un mélange de deux ou plusieurs composés différant par la nature du cation alcalin et/ou dérivant de deux ou plusieurs polyphénols distincts.

Selon un mode de réalisation préféré de la présente invention, on utilise un sel alcalin de diphénol ou un mélange de deux ou plusieurs sels alcalins de diphénols pouvant différer par la nature du cation alcalin et/ou dérivant de deux ou plusieurs diphénols distincts. On utilise, de préférence, des sels de sodium ou de potassium de un ou plusieurs diphénols, en particulier les sels de diphénols choisis dans le groupe constitué par: le bisphénol-A, le bisphénol-F, le bis(hydroxy-4 phényl)-2,2 dichloro-1,1 éthylène et le bis(dibromo-3,5, hydroxy-4 phényl)-2,2 propane.

Selon une variante préférée de l'invention, on utilise les sels alcalins du bisphénol-A et/ou du bisphénol-F, et plus particulièrement, les sels de sodium ou de potassium.

Les sels de sodium du bisphénol-A et/ou du bisphénol-F conviennent particulièrement bien à la mise en oeuvre de la présente invention. On préfère tout particulièrement le sel de sodium du bisphénol-A.

La quantité de cataliseur à mettre en oeuvre n'est pas critique.

On observe de bons résultats lorsqu'on engage au moins 0,05 groupes actifs équivalent de formule (I) pour 100 équivalents de groupement OM en provenance des sels alcalins de polyphénols. Il n'y a pas d'avantage particulier à en utiliser plus de 5 équivalents pour 100 équivalents de groupements (OM).

De préférence, on utilise de 0,25 à 2,5 équivalents de formule (I) pour 100 équivalents de groupements OM.

Selon une variante préférée de la présente invention, lorsqu'on met en oeuvre des sels potassiques de polyphénols, on utilise conjointement des catalyseurs dont les groupes fonctionnels sont représentés par la formule (I) dans laquelle $R_1$ à $R_4$, $R_6$ et $R_7$ représentent un atome d'hydrogène, $R_5$ et $R_8$, identiques représentent un atome d'hydrogène ou, de préférence un radical alkyle ayant de 1 à 4 atomes de carbone, m, n et p sont supérieurs ou égaux à 2.

Selon une variante encore plus avantageuse, on met en oeuvre des sels sodiques de polyphénols et des catalyseurs dont les groupes fonctionnels sont représentés par la formule (I) dans laquelle $R_1$ à $R_4$, $R_6$ et $R_7$ représentent un atome d'hydrogène, $R_5$ et $R_8$, identiques représentent un atome d'hydrogène ou, de préférence un radical alkyle ayant de 1 à 4 atomes de carbone m, n et p sont supérieurs ou égaux à 1.

De nombreux halo-1 époxy-2,3 alcanes conviennent à la mise en oeuvre de la présente invention. On peut citer à titre d'exemple, le chloro-1 époxy-2,3 propane (plus couramment appelé épichlorhydrine), le bromo-1 époxy-2,3 propane, le chloro-1 époxy-2,3 butane, le chloro-1 méthyl-2 époxy-2,3 propane, ainsi que leurs mélanges.

Selon une variante préférée de la présente invention, on utilise l'épichlorhydrine.

Par milieu sensiblement aprotique on entend un milieu ne renfermant pratiquement pas de protons, à l'exception de ceux susceptibles de provenir de certains catalyseurs.

Selon un premier mode de réalisation de la présente invention, on fait réagir au moins une mole de halo-1 époxy-2,3 alcane par équivalent-gramme de groupements OM en provenance d'au moins un sel alcalin de polyphénol chargé initialement, en milieu sensiblement aprotique.

On n'observe pas d'avantage particulier lorsqu'on opère avec plus de 13 moles de halo-1 époxy-2,3 alcane par équivalent-gramme de groupements OM. On opère de préférence avec 1 à 5 moles de halo-1 époxy-2,3 alcane par équivalent-gramme de groupements OM.

Les sels alcalins de polyphénols étant insolubles dans les halo-1 époxy-2,3 alcanes, il est souhaitable d'opérer avec une agitation suffisante.

Selon un deuxième mode de réalisation de la présente invention, on peut introduire le (ou les) sel(s) alcalin(s) progressivement, par exemple en un nombre variable de fractions égales ou différentes, plus ou moins espacées dans le temps, ou en continu.

La température de réaction n'est pas critique et elle est généralement comprise entre 50 et 150° C. Au-dessous de 50° C la réaction est relativement lente, et au-dessus de 150° C on risque de dégrader plus ou moins la résine obtenue.

On opère de préférence à une température comprise entre 70 et 120° C, environ.

L'addition au milieu réactionnel d'au moins un composé organique aprotique et de préférence polaire, apporte un effet favorable, même lorsque des quantités de l'ordre de quelques pourcents en poids sont présentes dans le milieu réactionnel. A titre d'exemple de composés organiques utilisables dans le cadre du présent procédé, on peut citer la N-méthylpyrrolidone, le diméthylformamide, le diméthylacétamide, le diméthylsulfoxyde, le dipropylsulfoxyde, la tétraméthylènesulfone, l'acétonitrile, le propionitrile, le benzonitrile, le sulfure d'éthylène. Exceptionnellement, certains de ces composés rendront le milieu homogène, mais ceci n'est en aucun cas nécessaire.

Lorsqu'on opère en présence d'un tel composé organique, on obtient de bons résultats même lorsque la quantité de halo-1 époxy-2,3 alcane est inférieure ou égale à 3 Moles par équivalent-gramme de groupements OM, ce qui constitue un mode de réalisation du présent procédé, particulièrement avantageux.

En général, une quantité de composé organique anhydre, aprotique et polaire de l'ordre de 10% en poids est ajoutée au milieu réactionnel, cette quantité représente de préférence, au moins 20% (en poids) du milieu réactionnel. On peut en utiliser des quantités plus importantes, mais au-delà de 80% (en poids) on n'observe pas d'avantage particulier.

Selon une variante avantageuse de l'invention, on opère avec 10 à 80% en poids d'acétonitrile, et de préférence au moins 30% en poids d'acétonitrile.

On peut également opérer sous pression autogène ou sous une pression d'azote pouvant atteindre 20 bars.

Un avantage procuré par la mise en oeuvre, selon la présente invention, d'un catalyseur supporté constitué par un support polymère organique réticulé et par une pluralité de groupes fonctionnels, fixés sur ce support et représentés par la formule (I) réside dans le fait qu'en fin de réaction, on peut séparer aisément ledit catalyseur du milieu réactionnel. En effet cette séparation peut se faire par simple décantation et filtration. En outre, il est possible de recycler une ou plusieurs fois ce catalyseur sans observer de perte sensible en efficacité; en fin de réaction, on récupère le catalyseur par tout moyen approprié et on élimine par exemple par un lavage à l'eau, l'halogénure alcalin co-produit. Il n'est pas nécessaire de purifier et/ou de sécher ce catalyseur susceptible de contenir des traces de produit, voire de réactifs non transformés et le cas échéant de solvant. D'autre part, la demanderesse a constaté que les résines préparées par le présent procédé, sont peu colorées et que leurs caractéristiques n'évoluent pas de manière significative au cours du temps. En d'autres termes, la qualité des résines est améliorée et ces résines n'évoluent pratiquement pas au stockage.

Le procédé selon l'invention convient notamment à la préparation des résines époxydes liquides, à partir d'épichlorhydrine et de sels alcalins du bisphénol-A et/ou du bisphénol-F, et plus particulièrement à partir du sel disodique du bisphénol-A, en présence d'un catalyseur supporté constitué par un copolymère chlorométhyle du styrène et du divinylbenzène, dont le taux de réticulation est inférieur à 10% et dont le taux de chlore est de l'ordre de 0,5 à 7 meq/g, et par une pluralité de groupes fonctionnels fixés sur le dit support et ayant la formule:

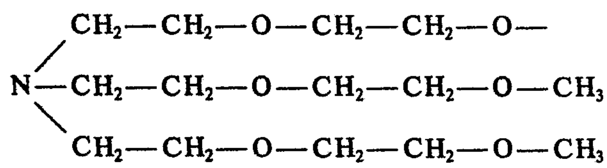

ou

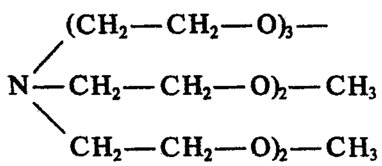

Ce type de résine a généralement une viscosité mesurée à 25°C inférieure ou égale à 150 poises (15 Pa · s) et un taux d'époxyde pour 100 g de l'ordre de 0,5.

Les exemples ci-après illustrent l'invention sans en limiter le domaine ou l'esprit.

## Exemples

### Préparation du sel disodique du bisphénol-A

(appelé »bisphénate de sodium« dans les exemples ci-après).

A une solution de 310 g (7,74 Moles) de soude et de 2407 $cm^3$ de méthanol on ajoute 883,5 g (3,87 moles) de bisphénol-A. On chauffe le mélange à reflux pendant 30 mn, puis on distille environ 70% du méthanol. A la pâte restante on ajoute 3000 $cm^3$ d'acétone et on filtre la suspension de sel disodique du bisphénol-A. Après lavage le sel est séché en étuve sous 5 mm Hg durant 24 heures à 50°C (premier lot) ou à 25°C (deuxième lot).

### Préparation des catalyseurs:

a) Préparation de la tris(dioxa-3,6 octyl) amine:

(catalyseur utilisé dans l'essai témoin »b« ci-après)

Ce catalyseur est préparé à partir de chlorhydrate de tris(chloro-2 éthyl) amine et d'éthoxy-2

éthanolate de sodium comme suit:

Dans un ballon tricol d'un litre, muni d'un agitateur mécanique, d'un thermomètre, d'un réfrigérant, on introduit 450 g d'éthoxy-2 éthanol (5 moles). On ajoute 23 g de sodium (1 mole), en 3 heures en maintenant la température de mélange à 40° C.

Au mélange ainsi obtenu, on ajoute 51,6 de chlorhydrate de tris(chloro-2 éthyl)amine (soit 0,215 mole). On chauffe alors le mélange à reflux de l'éthoxy-2-éthanol pendant 12 heures puis on distille le solvant sous pression réduite. L'éthoxy-2 éthanolate de sodium en excès est neutralisé par addition de 12 $cm^3$ d'HCl aqueux (10 N).

Le chlorure de sodium formé est filtré et la solution est distillée. La tris(dioxa-3,6 octyl)amine distille entre 200° C et 210° C sous 1 mm Hg.

b) Préparation du N(hydroxy-8', dioxa-3',6' octyl) aza-8 tétraoxa-2,5,11,14 pentadécane:

(aminoalcool utilisé dans la préparation du catalyseur n° 2 ci-après)

de formule:

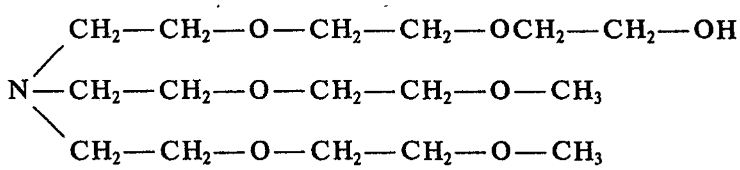

Dans un ballon tétracol de 2 litres, équipé d'une agitation, d'une arrivée d'hydrogène, d'une colonne et d'un condenseur pour recueillir l'eau, on charge:

- 890 g (6 moles) de triéthylène glycol,
- 160 g de Ni Raney déshydraté,
- 442 g (2 moles) d'aza-8 tétraoxa-2,5,11,14 pentadécane.

On agite sous un courant d'hydrogène (1 l/mn) et on chauffe 3 heures à 180° C. Après filtration du nickel et distillation pour éliminer le triéthylène glycol, on récupère 508 g de l'aminoalcool attendu, dont le point d'ébullition sous 0,3 mm Hg est 193° C.

NB: le N(hydroxy-5', oxa-3' pentyl)aza-8, tétraoxa-2,5,11,14 pentadécane, aminoalcool utilisé dans la préparation du catalyseur n° 1 ci-après, est préparé de manière similaire.

c) Préparation du catalyseur n° 1

(catalyseur utilisé dans l'exemple 1 ci-après).

Dans un réacteur tricol de 250 ml, équipé d'un agitateur magnétique, d'un réfrigérant ascendant et d'une arrivée d'azote on introduit successivement 300 $cm^3$ de toluène anhydre, 36,05 g de N(hydroxy-5', oxa-3' pentyl) aza-8, tétraoxa-2,5,11,14 pentadécane et 2,41 g sodium. Après chauffage à 60° C pendant 20 heures et à 90° C pendant 4 heures, le sodium a totalement réagi. On refroidit alors à 60° C puis on introduit 52 g de polystyrène reticulé par 2% de divinylbenzène et contenant 1,3 meq chlore/g.

Après 40 heures à 60° C sous atmosphère d'azote, le polymère est refroidi. Il est alors filtré et lavé à l'eau (pour en éliminer les sels inclus) et au méthanol. On sèche ensuite le produit obtenu sous vide à 50° C.

On obtient ainsi 66 g d'un amino-éther greffé sur le polystyrène dont la formule générale est la suivante:

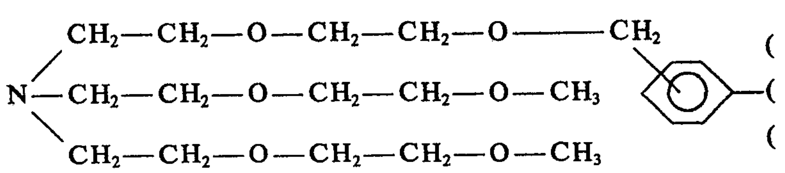

Le taux de greffage est de 75%. D'après le taux d'azote, ce composé renferme 0,929 milliéquivalent (meq) de groupes fonctionnels par gramme.

d) Préparation du catalyseur n° 2:

(catalyseur utilisé dans l'exemple 2 ci-après)

Dans un réacteur tricol de 250 ml, équipé d'un agitateur magnétique, d'un réfrigérant ascendant et d'une arrivée d'azote on introduit successivement 100 $cm^3$ de toluène anhydre, 9,1 g de N(hydroxy-8' dioxa-3',6', octyl) aza-8 tétraoxa-2,5,11,14 pentadécane et 0,5 g de sodium métal. Après 7 h à 60° C sous agitation, le sodium a totalement disparu. On introduit alors 5 g de polystyrène réticulé par 2% de divinyl benzène et contenant 4 $10^{-3}$ groupement chlorométhyl par gramme de polymère (4 meq de chlore/g).

Le mélange est chauffé à 60° C pendant 48 heures sous atmosphère d'azote. Après refroidissement le polymère est filtre et lavé à l'eau (pour éliminer les sels inclus) puis au méthanol.

Le produit est ensuite séché sous vide à 50° C.

On obtient ainsi 8,8 g d'un amino éther greffé sur le polystyrène dont la formule générale est la suivante:

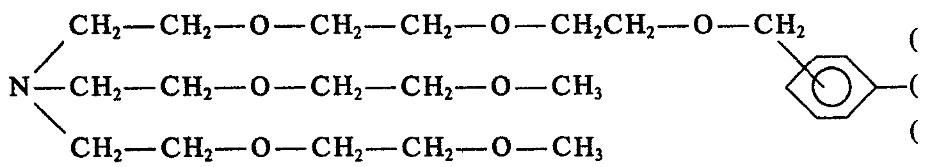

**Le taux de greffage est de 64%**

Dans les essais témoins et dans l'exemple 1 ci-après, on a utilisé du bisphénate de sodium en provenance du premier lot.

Essai témoin (a):

Cet essai illustre la préparation d'une résine époxyde à partir de bisphénate de sodium et d'épichlorhydrine en l'absence de catalyseur.

Dans un réacteur en verre équipé d'un agitateur central à ancre, d'un réfrigérant à boules et d'un thermomètre, on charge 92,5 g (1 mole) d'épichlorydrine, 56,4 $cm^3$ d'acétonitrile. On porte la masse à 60° C et on ajoute 54,4 g (0,2 mole) de bisphénate de sodium anhydre. On obtient une suspension que l'on porte à 83° C (reflux de l'acétonitrile) pendant 1 heure. On filtre la suspension et on évapore le filtrat sous pression réduite (20 mm Hg) en chauffant jusqu'à 140° C.

On récupère 27,2 g d'une résine dont les caractéristiques sont les suivantes:

- viscosité mesurée à 25° C = 240 poises (24 Pa · s)
- taux d'époxyde pour 100 g = 0,512
- Coloration = 7 garner

Cette résine n'évolue pas au stockage; après 100 jours la viscosité (à 25° C) est de 255 poises (25,5 Pa.s).

Essai témoin (b)

Cet essai illustre la préparation d'une résine époxyde à partir de bisphénate de sodium et d'épichlorydrine en présence d'un catalyseur non supporté.

Dans l'appareillage décrit ci-avant, on introduit 92,5 g (1 mole) d'épichlorhydrine, 57,7 $cm^3$ d'acétonitrile, 3,65 g (10 mMol) de tris (dioxa-3,6 octyl)amine. On porte la masse à 60° C et on ajoute 54,4 g (0,2 mole) de bisphénate de sodium anhydride. On obtient une suspension que l'on porte à 83° C (reflux de l'acétonitrile) pendant 1 heure. On filtre la suspension et on évapore le filtrat sous pression réduite (20 mm Hg) en chauffant jusqu'à 140° C.

On récupère 65,4 g d'une résine dont les caractéristiques sont les suivantes:

- viscosité mesurée à 25° C = 85 poises (8,5 Pa · s)
- taux d'époxyde pour 100 g = 0,506
- coloration = 13 garner
- basicité (déterminée par dosage à l'acide perchlorique) = 0,1550 meq/g

Cette résine évolue au stockage, après 10 jours la viscosité (à 25° C) est de 2480 poises (248 Pa · s).

Exemple 1

On reproduit l'essai témoin »b« ci-avant mais en utilisant 10,76 g du catalyseur n° 1 décrit précédemment, soit 10 meq de groupes fonctionnels; le volume d'acétonitrile est de 61 $cm^3$.

On obtient 48,8 g d'une résine liquide dont les caractéristiques sont les suivantes:

- viscosité mesurée à 25° C = 120 poises (12 Pa · s)
- taux d'époxide pour 100 g = 0,537
- coloration = 5 garner
- basicité (dérminée par dosage à l'acide perchlorique) = 0,0065 meq/g

Cette résine est stable au stockage; après 10 jours la viscosité (à 25° C) est de 120 poises (12 Pa · s).

En fin de réaction, le catalyseur (insoluble dans le milieu) est récupéré. On le lave avec du méthanol pour dissoudre le bisphénate de sodium qu'il est susceptible de renfermer, et avec de l'eau pour dissoudre le chlorure de sodium qui le souille. Le catalyseur est alors séché à 50° C sous vide; il peut être utilisé alors dans une nouvelle opération.

Le bisphénate de sodium utilisé dans les exemples ci-après provient du deuxième lot défini précédemment.

Exemple 2

Dans l'appareillage et selon le mode opératoire décrit précédemment on prépare une résine à partir de 92,5 g (1 mole) d'épichlorhydrine, 66 $cm^3$ d'acétonitrile, 68,5 g du catalyseur N° 2 décrit précédemment soit 8,85 meq de groupes fonctionnels, et 73,9 g (0,2 moles) de bisphénate de sodium (sensiblement anhydre).

On récupère 40,6 g d'une résine dont les caractéristiques sont les suivantes:

- viscosité mesurée à 25° C = 96 poises (9,6 Pa · s)
- taux d'époxyde pour 100 g = 0,539
- coloration = 11 garner

La viscosité (à 25° C) est de 93 poises (9,3 Pa · s) après 10 jours de stockage.

Exemple 3

Dans l'appareillage et selon le mode opératoire décrit précédemment on prépare une résine à partir de 69,4 g (0,75 mole) d'épichlorhydrine, 50 $cm^3$ d'acétonitrile, 6,58 g de catalyseur récupéré à l'exemple 2, soit 6,64 meq de groupes fonctionnels, et 55,2 g (0,15 mole) de bisphénate de sodium (sensiblement anhydre).

On obtient 30 g d'une résine dont les caractéristiques sont les suivantes:

- viscosité mesurée à 25° C = 91 poises (9,1 Pa · s)
- taux d'époxyde pour 100 g = 0,548
- coloration = 6 garner

La viscosité (à 25° C) est de 96 poises (9,6 Pa · s) après 10 jours de stockage.

Exemple 4

On reproduit l'exemple 3 ci-avant, en utilisant 9,08 g de catalyseur (soit 6,64 meq de groupes fonctionnels) récupéré à l'exemple 3.

On obtient 29 g d'une résine dont les caractéristiques sont les suivantes:

- viscosité mesurée à 25° C = 90 poises (9 Pa · s)
- taux d'époxyde pour 100 g = 0, 543
- coloration = 7 garner

La viscosité (à 25° C) est de 98 poises (9,8 Pa.s), après 10 jours de stockage.

Exemple 5

Dans l'appareillage et selon le mode opératoire décrit précédemment on prépare une résine à partir de 46,3 g (0,5 mole) d'épichlorhydrine, 31 $cm^3$ d'acétonitrile, 8,57 g de catalyseur récupéré à l'exemple 4 soit 4,42 meq de groupes fonctionnels, et 36,9 g (0,1 mole) de bisphénate de sodium (sensiblement anhydre).

On obtient 19 g d'une résine dont les caractéristiques sont les suivantes:

- viscosité mesurée à 25° C = 91 poises (9,1 Pa · s)
- taux d'époxyde pour 100 g = 0,538
- coloration = 6 garner

La viscosité (à 25° C) est de 92 poises (9,2 Pa.s), après 10 jours de stockage.

Exemple 6

On reproduit l'exemple 5 ci-avant, en utilisant 5,38 g du catalyseur n° 1, dont la préparation est décrite précédemment, soit 5 meq de groupes fonctionnels.

On obtient 25,7 g d'une résine dont les caractéristiques sont les suivantes:

- viscosité mesurée à 25° C = 110 poises (11 Pa · s)
- taux d'époxyde pour 100 g = 0,557
- coloration = 5 garner

La viscosité (à 25° C) est de 110 poises (11 Pa · s), après 10 jours de stockage.

**Revendications**

1. Procédé de préparation de polyéthers glycidiques de polyphénols par réaction en milieu anhydre et sensiblement aprotique, d'au moins un sel alcalin de polyphénol sur au moins un halo-1 époxy-2,3 alcane caractérisé en ce qu'on opère en présence d'un catalyseur supporté constitué par un support polymère organique réticulé et par une pluralité de groupes fonctionnels (groupes actifs du catalyseur), fixés sur ledit support et ayant pour formule générale:

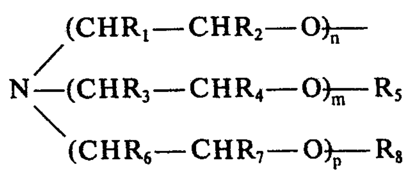

(I)

dans laquelle $R_1$, $R_2$, $R_3$, $R_4$, $R_6$ et $R_7$, identiques ou différents, sont chacun un atome d'hydrogène ou un radical alkyle ayant de 1 à 4 atomes de carbone, $R_5$ et $R_8$, identiques ou différents, représentent un atome d'hydrogène, un radical alkyle ou cycloalkyle ayant de 1 à 12 atomes de carbone un radical phényle ($C_6H_5-$), un radical $C_6H_5 - C_q H_{2q} -$ ou $C_q H_{2q+1} - C_6H_5 -$ avec q compris entre 1 et 12 ($1 \leq q \leq 12$), et dans laquelle n, m et p, identiques ou différents, sont supérieurs ou égaux à 1 et inférieurs ou égaux à 10.

2. Procédé selon la revendication 1, caractérisé en ce que les groupes fonctionnels du catalyseur sont choisis parmi les groupes de formule (I) dans laquelle $R_1$, $R_2$, $R_3$, $R_4$, $R_6$ et $R_7$, identiques ou différents, représentent un atome d'hydrogène ou un radical méthyle et $R_5$ et $R_8$, identiques ou différents, représentent un atome d'hydrogène ou un radical alkyle ayant de 1 à 4 atomes de carbone.

3. Procédé selon la revendication 1 ou 2, caractérisé en ce que les groupes fonctionnels du catalyseur sont représentés par la formule I dans laquelle n, m et p identiques ou différents sont supérieurs ou égaux à 1 et inférieurs ou égaux à 6.

4. Procédé selon l'une quelconque des revendications précédentes, caractérisé en ce que le support dérive d'un polymère organique réticulé comportant des groupements substituables par les groupements fonctionnels de formule I.

5. Procédé selon l'une quelconque des revendications précédentes, caractérisé en ce que le support dérivé d'un polymère dont le taux de réticulation est inférieur à 10%, et de préférence, inférieur à 5%.

6. Procédé selon l'une quelconque des revendications précédentes, caractérisé en ce que le support dérive d'un polystyrène réticulé par le divinylbenzène.

7. Procédé selon la revendication 5 ou 6, caractérisé en ce que le support dérive d'un polystyrène chloro ou bromométhylé.

8. Procédé selon la revendication 7, caractérisé en ce que le pourcentage de noyaux benzéniques du

polystyrène portant un groupement chloro ou bromo méthyle est supérieur à 5%, et de préférence, supérieur à 10%.

9. Procédé selon la revendication 8, caractérisé en ce que le polystyrène a un taux de brome ou de chlore compris entre 0,5 et 7 milliéquivalents de brome ou de chlore par gramme.

10. Procédé selon l'une quelconque des revendications 5 à 9, caractérisé en ce que le catalyseur est représenté par la formule

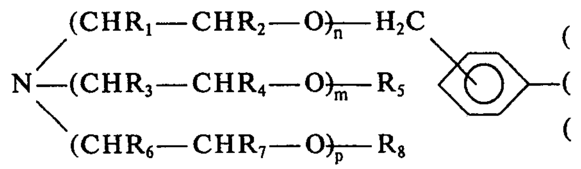

dans laquelle $R_1$ à $R_8$, n, m et p ont la signification donnée

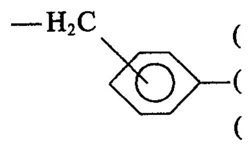

dérive du polystyrène chloro du bromo méthylé de formule

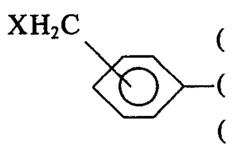

X représentant un atome de chlore ou de brome.

11. Procédé selon la revendication 1 caractérisé en ce catalyseur est représenté par la formule

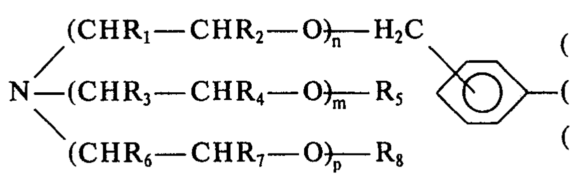

dans laquelle $R_1$ à $R_4$, $R_6$ et $R_7$ représentent l'hydrogène, $R_5$ et $R_8$, identiques représentent l'hydrogène ou de préférence un radical alkyle, ayant de 1 à 4 atomes de carbone, m, n et p étant supérieurs ou égaux à 1, et inférieurs ou égaux à 6,

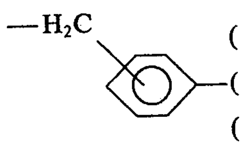

dérive d'un polystyrène chloro ou bromométhylé, réticulé par le divinylbenzène, le taux de réticulation étant inférieur à 10% et le taux de chlore ou de brome étant compris entre 0,8 et 7 meq/g.

12. Procédé selon l'une quelconque des revendications précédentes, caractérisé en ce que le polyphénol est choisi dans le groupe constitué par le bisphénol-A, le bisphénol-F, le bis(hydroxy-4 phényl)-2,2 dichloro-1,1 éthylène et le bis(dibromo-3,5, hydroxy-4 phényl)-2,2 propane.

13. Procédé selon la revendication 12, caractérisé en ce que le polyphénol est choisi dans le groupe constitué par le bisphénol-A, le bisphénol-F et leurs mélanges.

14. Procédé selon la revendication 13, caractérisé en ce que le polyphénol est le bisphénol-A.

15. Procédé selon l'une quelconque des revendications précédentes, caractérisé en ce que le halo-1 époxy-2,3 alcane est l'épichlorydrine.

16. Procédé selon l'une quelconque des revendications précédentes, caractérisé en ce que l'on opère en présence de 1 à 13 moles, et de préférence de 1 à 5 moles de halo-1 époxy-2,3 alcane par équivalent-gramme de groupements OM en provenance d'un sel alcalin de polyphénol.

17. Procédé selon l'une quelconque des revendications précédentes, caractérisé en ce que l'on opère en présence de 0,05 à 5 équivalents, et de préférence de 0,25 à 2,5 équivalents de groupements fonctionnels de formule (I) pour 100 équivalents de groupements OM en provenance d'un sel alcalin de polyphénol.

18. Procédé selon l'une quelconque des revendications précédentes, caractérisé en ce que l'on opère en présence d'un composé organique aprotique polaire choisi dans le groupe comprenant l'acétonitrile, le diméthylformamide, le diméthylacétamide, le diméthylsulfoxyde, le dipropylsulfoxyde, le propionitrile, le benzonitrile, le sulfure d'éthylène, la N-méthylpyrrolidone et la tétraméthylènesulfone.

19. Procédé selon la revendication 18, caractérisé en ce que l'on opère en présence d'acétonitrile.

20. Procédé selon l'une quelconque des revendication précédentes, caractérisé en ce que la température de réaction est comprise entre 50 et 150° C, et de préférence, entre 70 et 120° C.

## Patentansprüche

1. Verfahren zur Herstellung von Glycidylpolyethern von Polyphenolen durch Umsetzung mindestens eines Alkalisalzes eines Polyphenols mit mindestens einem 1-Halogen-2,3-epoxyalkan in wasserfreiem und im wesentlichen aprotischen Medium, dadurch gekennzeichnet, daß man in Gegenwart eines Trägerkatalysators arbeitet, der aus einem vernetzten organischen Polymeren als Träger und mehreren funktionellen Gruppen (aktive Gruppen des Katalysators) besteht, die an den Träger gebunden sind und der allgemeinen Formel

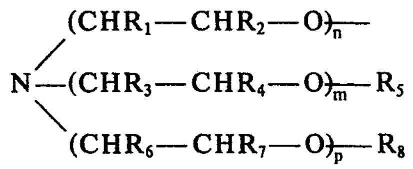

(I)

entsprechen, in der $R_1$, $R_2$, $R_3$, $R_4$, $R_6$ und $R_7$ gleich oder verschieden sind und jeweils für ein Wasserstoffatom oder für eine Alkylgruppe mit 1 bis 4 Kohlenstoffatomen stehen, $R_5$ und $R_8$ gleich oder verschieden sind und für ein Wasserstoffatom, eine Alkyl- oder Cycloalkylgruppe mit 1 bis 12 Kohlenstoffatomen, eine Phenylgruppe ($C_6H_5-$), oder für eine Gruppe $C_6H_5 - C_q H_{2q} -$ oder $C_q H_{2q+1} - C_6H_5-$ stehen, in der q 1 bis 12 bedeutet ($1 \leqq q \leqq 12$) und wobei n, m und p gleich oder verschieden sind und eine Zahl von 1 bis 10 bedeuten.

2. Verfahren nach Anspruch 1, dadurch gekennzeichnet, daß die funktionellen Gruppen des Katalysators unter den Gruppen der Formel (I) ausgewählt werden, in der $R_1$, $R_2$, $R_3$, $R_4$, $R_6$ und $R_7$, die gleich oder verschieden sind, für ein Wasserstoffatom oder eine Methylgruppe und $R_5$ und $R_8$, die gleich oder verschieden sind, für ein Wasserstoffatom oder eine Alkylgruppe mit 1 bis 4 Kohlenstoffatomen stehen.

3. Verfahren nach Anspruch 1 oder 2, dadurch gekennzeichnet, daß die funktionellen Gruppen des Katalysators durch die Formel (I) wiedergegeben werden, in der n, m und p gleich oder verschieden sind und (jeweils) eine Zahl von 1 bis 6 bedeuten.

4. Verfahren nach einem der vorangehenden Ansprüche, dadurch gekennzeichnet, daß der Träger sich von einem vernetzten organischen Polymeren ableitet, das Gruppen aufweist, die durch die funktionellen Gruppen der Formel (I) ersetzt werden können.

5. Verfahren nach einem der vorangehenden Ansprüche, dadurch gekennzeichnet, daß der Träger sich von einem Polymeren ableitet, dessen Vernetzungsgrad unter 10% und vorzugsweise unter 5% liegt.

6. Verfahren nach einem der vorangehenden Ansprüche, dadurch gekennzeichnet, daß der Träger sich von einem mit Divinylbenzol vernetzten Polystyrol ableitet.

7. Verfahren nach Anspruch 5 oder 6, dadurch gekennzeichnet, daß der Träger sich von einem chlor- oder brommethylierten Polystyrol ableitet.

8. Verfahren nach Anspruch 7, dadurch gekennzeichnet, daß der prozentuale Anteil von Benzolkernen des Polystyrols, die eine chlor- oder brommethylierte Gruppe tragen, mehr als 5% und vorzugsweise mehr als 10% ausmacht.

9. Verfahren nach Anspruch 8, dadurch gekennzeichnet, daß das Polystyrol einen Brom- oder Chlorgehalt aufweist, der 0,5 bis 7 mÄq Brom oder Chlor je Gramm beträgt.

10. Verfahren nach einem der Ansprüche 5 bis 9, dadurch gekennzeichnet, daß der Katalysator durch die Formel

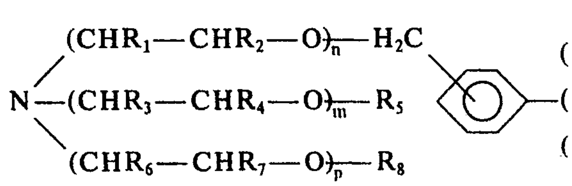

wiedergegeben wird, in der $R_1$ bis $R_8$, n, m und p die oben angegebene Bedeutung haben,

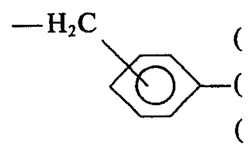

sich von chlor- oder brommethyliertem Polystyrol der Formel

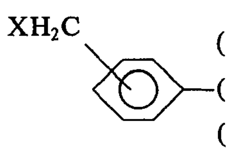

ableitet, wobei X ein Chlor- oder Bromatom bedeutet.

11. Verfahren nach Anspruch 1, dadurch gekennzeichnet, daß der Katalysator durch die Formel

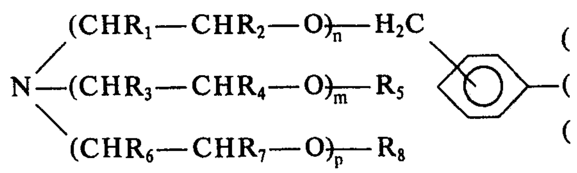

wiedergegeben wird, in der $R_1$ bis $R_4$, $R_6$ und $R_7$ Wasserstoff bedeuten, $R_5$ und $R_8$ gleich sind und für Wasserstoff oder vorzugsweise eine Alkylgruppe mit 1 bis 4 Kohlenstoffatomen stehen und m, n und p eine Zahl von 1 bis 6 bedeuten,

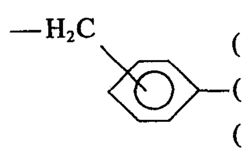

sich von einem chlor- oder brommethylierten Polystyrol ableitet, das durch Divinylbenzol vernetzt ist, wobei der Vernetzungsgrad weniger als 10% beträgt und der Gehalt an Chlor oder Brom 0,8 bis 7 mÄq/g beträgt.

12. Verfahren nach einem der vorangehenden Ansprüche, dadurch gekennzeichnet, daß das Polyphenol aus der Gruppe, bestehend aus Bisphenol A, Bisphenol F, 2,2-Bis(4-hydroxyphenyl)-1,1-dichlorethylen und 2,2-Bis(3,5-dibrom-4-hydroxyphenyl)propan ausgewählt wird.

13. Verfahren nach Anspruch 12, dadurch gekennzeichnet, daß das Polyphenol aus der Gruppe, bestehend aus Bisphenol A, Bisphenol F und deren Gemischen ausgewählt wird.

14. Verfahren nach Anspruch 13, dadurch gekennzeichnet, daß das Polyphenol das Bisphenol A ist.

15. Verfahren nach einem der vorangehenden Ansprüche, dadurch gekennzeichnet, daß das 1-Halogen-2,3-epoxyalkan Epichlorhydrin ist.

16. Verfahren nach einem der vorangehenden Ansprüche, dadurch gekennzeichnet, daß man in Gegenwart von 1 bis 13 mol, vorzugsweise 1 bis 5 mol 1-Halogen-2,3-epoxyalkan je gÄq Gruppen OM, die von einem Polyphenol-alkalisalz stammen, arbeitet.

17. Verfahren nach einem der vorangehenden Ansprüche, dadurch gekennzeichnet, daß man in Gegenwart von 0,05 bis 5 Äq und vorzugsweise 0,25 bis 2,5 Äq funktionelle Gruppen der Formel (I) auf 100 Äq Gruppen OM, die von einem Polyphenol-alkalisalz stammen arbeitet.

18. Verfahren nach einem der vorangehenden Ansprüche, dadurch gekennzeichnet, daß man in Gegenwart einer organischen aprotischen polaren Verbindung arbeitet, die aus der Gruppe, bestehend aus Acetonitril, Dimethylformamid, Dimethylacetamid, Dimethylsulfoxid, Dipropylsulfoxid, Propionitril, Benzonitril, Ethylensulfid, N-Methylpyrrolidon und Tetramethylensulfon ausgewählt ist.

19. Verfahren nach Anspruch 18, dadurch gekennzeichnet, daß man in Gegenwart von Acetonitril arbeitet.

20. Verfahren nach einem der vorangehenden Ansprüche, dadurch gekennzeichnet, daß die Reaktionstemperatur 50 bis 150° C, vorzugsweise 70 bis 120° C beträgt.

**Claims**

1. Process for the preparation of glycidyl polyethers of polyphenols by reacting, in an anhydrous and substantially aprotic medium, at least one alkali metal salt of a polyphenol with at least one 1-halo-2,3-epoxy-alkane, characterised in that the reaction is carried out in the presence of a supported catalyst consisting of a cross-linked organic polymeric support and a plurality of functional groups (active groups of the catalyst) fixed to the said support and having the general formula:

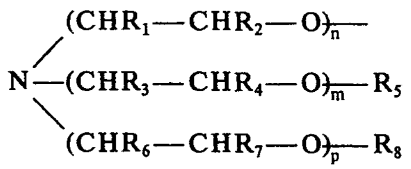

(I)

in which $R_1$, $R_2$, $R_3$, $R_4$, $R_6$ and $R_7$, which may be identical or different, are each a hydrogen atom or an alkyl radical having 1 to 4 carbon atoms, $R_5$ and $R_8$, which may be identical or different, represent a hydrogen atom, an alkyl or cycloalkyl radical having from 1 to 12 carbon atoms, a phenyl radical ($C_6H_5$—) or a $C_6H_5 - C_qH_{2q}-$ or $C_qH_{2q+1} - C_6H_5 -$ radical with q between 1 and 12 ($1 \leq q \leq 12$), n, m and p, which may be identical or different, being greater than or equal to 1 and less than or equal to 10.

2. Process according to claim 1, characterised in that the functional groups of the catalyst are chosen from amongst the groups of the formula (I) in which $R_1$, $R_2$, $R_3$, $R_4$, $R_6$ and $R_7$, which may be identical or different, represent a hydrogen atom or a methyl radical and $R_5$ and $R_8$, which may be identical or different, represent a hydrogen atom or an alkyl radical having from 1 to 4 carbon atoms.

3. Process according to claim 1 or 2, characterised in that the functional groups of the catalyst are represented by the formula I, in which n, m and p, which may be identical or different, are greater than or equal to 1 and less than or equal to 6.

4. Process according to any one of the preceding claims, characterised in that the support is derived from a crosslinked organic polymer containing groups which can be replaced by functional groups of the formula I.

5. Process according to any one of the preceding claims, characterised in that the support is derived from a polymer whose degree of crosslinking is less than 10% and preferably less than 5%.

6. Process according to any one of the preceding claims, characterised in that the support is derived from a polystyrene crosslinked with divinylbenzene.

7. Process according to claim 5 or 6, characterised in that the support is derived from a chloromethylated or bromomethylated polystyrene.

8. Process according to claim 7, characterised in that the percentage of benzene rings of the polystyrene which carry a chloromethyl or bromomethyl group is greater than 5% and preferably greater than 10%.

9. Process according to claim 8, characterised in that the polystyrene has a bromine or chlorine content of between 0,5 and 7 milliequivalents of bromine or chlorine per gram.

10. Process according to any one of claims 5 to 9, characterised in that the catalyst is represented by the formula

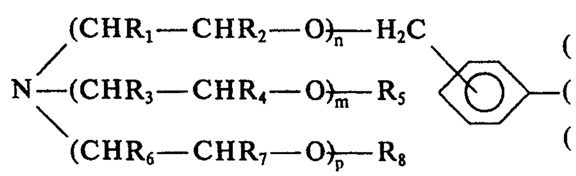

in which $R_1$ to $R_8$, n, m and p have the meaning given, and

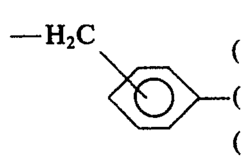

is derived from chloromethylated or bromomethylated polystyrene of the formula

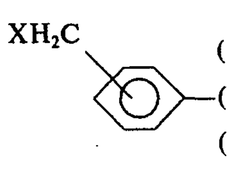

where X represents a chlorine or bromine atom.

11. Process according to claim 1, characterised in that the catalyst is represented by the formula

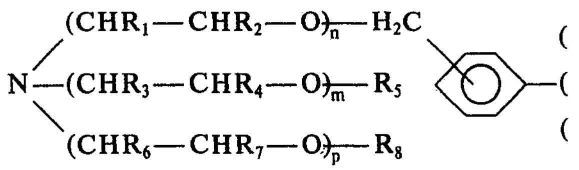

in which $R_1$ to $R_4$, $R_6$ and $R_7$ represent hydrogen, $R_5$ and $R_8$, which are identical, represent hydrogen or, preferably, an alkyl radical having from 1 to 4 carbon atoms, m, n and p being greater than or equal to 1 and less than or equal to 6, and

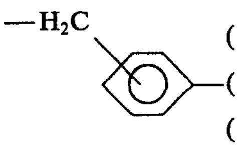

is derived from a chloromethylated or bromomethylated polystyrene crosslinked with divinylbenzene, the degree of crosslinking being less than 10% and the chlorine or bromine content being between 0.8 and 7 milliequivalents/g.

12. Process according to any one of the preceding claims, characterised in that the polyphenol is chosen from the group consisting of bisphenol-A, bisphenol-F, 2,2-bis-(4-hydroxyphenyl)-1,1-dichloroethylene and 2,2-bis-(3,5-dibromo-4-hydroxyphenyl)-propane.

13. Process according to claim 12, characterised in that the polyphenol is chosen from the group consisting of bisphenol-A, bisphenol-F and their mixtures.

14. Process according to claim 13, characterised in that the polyphenol is bisphenol-A.

15. Process according to any one of the preceding claims, characterised in that the 1-halo-2,3-epoxy-alkane is epichlorohydrin.

16. Process according to any one of the preceding claims, characterised in that it is carried out in the presence of 1 to 13 moles, preferably 1 to 5 moles, of 1-halo-2,3-epoxy-alkane per gram-equivalent of OM groups originating from an alkali metal salt of a polyphenol.

17. Process according to any one of the preceding claims, characterised in that it is carried out in the presence of 0.05 to 5 equivalents, and preferably of 0.25 to 2.5 equivalents, of functional groups of the formula (I) per 100 equivalents of OM groups originating from an alkali metal salt of a polyphenol.

18. Process according to any one of the preceding claims, characterised in that it is carried out in the presence of a polar aprotic organic compound chosen from the group comprising acetonitrile, dimethylformamide, dimethylacetamide, dimethylsulphoxide, dipropylsulphoxide, propionitrile, bensonitrile, ethylene sulphide, N-methylpyrrolidone and tetramethylenesulphone.

19. Process according to claim 18, characterised in that it is carried out in the presence of acetonitrile.

20. Process according to any one of the preceding claims, characterised in that the reaction temperature is between 50 and 150° C and preferably between 70 and 120° C.